# EUROPEAN PATENT APPLICATION

(11) **EP 3 646 857 A1**
(43) Date of publication of application: **06.05.2020**
(21) Application number: 18203946.1
(22) Date of filing: 01.11.2018
(51) Int. Cl.: A61K 9/48, A23L 33/135, A61K 35/74, A61K 35/741, A61K 35/745, A61K 35/747

(54) **COLONIC DELIVERY SYSTEM FOR MICROORGANISMS**

(71) Applicant: Evonik Operations GmbH, 45128 Essen (DE)
(72) Inventor: ENGEL, Andrea, 60599 Frankfurt (DE); NIEPOTH, Peter, 64823 Groß-Umstadt (DE); FORLIZZI, Ilaria, 64295 Darmstadt (DE)
(74) Representative: Evonik Patent Association

(57) **Abstract**

The invention discloses a colonic delivery system for microorganisms comprising a capsule comprising one or more bacterial or yeast strains, wherein the capsule comprises a coating comprising a polymer polymerized from 10 to 30 % by weight methyl methacrylate, 50 to 70 % by weight methyl acrylate and 5 to 15 % by weight methacrylic acid and uses thereof.

## Description

The current invention concerns a colonic delivery system for microorganisms comprising a capsule comprising one or more bacterial or yeast strains, wherein the capsule comprises a coating comprising a polymer polymerized from 10 to 30 % by weight methyl methacrylate, 50 to 70 % by weight methyl acrylate and 5 to 15 % by weight methacrylic acid.

The gastrointestinal microbiota modulate health and has therefore emerged as a target of interventions to improve the health of humans and animals. Microbiota-targeted strategies include the application of prebiotics, probiotics, and sometimes even fecal transplantations with the intention to modify the composition and activity of the microbiota. Probiotics are live microorganisms, which confer a health benefit on the host when administered in adequate amounts (FAO-WHO; Probiotics in food. Health and nutritional properties and guidelines for evaluation; FAO Food and Nutritional Paper 85, 2006). The most commonly investigated and commercially available probiotics are mainly microorganisms from species of genera *Lactobacillus* and *Bifidobacterium.* In addition, several others such as *Propionibacterium, Streptococcus, Bacillus, Enterococcus, Escherichia coli,* and yeasts are also used. Different bacterial strains of the same genus and species may exert different effects on the host.

The gut microflora is an essential component of the healthy human. Microbiota therapies are used to address medical states or conditions and to maintain the gut health. The colon is heavily populated with microbiota, whereas the stomach and the upper intestine display sparsely populated regions. Hence, from a physiological perspective, the colon is the preferred site of delivery for microbiota.

In order to deliver microbiota to the colon via the oral delivery route, they have to pass the stomach followed by the small intestine. The environment in the stomach, in particular the acidic pH conditions, are lethal to many microorganisms. The harsh conditions wipe out almost all bacteria during their passage through it. Studies evaluating strain viability before and after in vitro gastric experiments revealed that a variety of strains are highly sensitive to those acidic conditions. The respective strains would not show any viability when ingested, especially in a fasted state (Caillard and Lapointe, 2017). For this reason, formulations protecting bacteria from the harsh conditions in the stomach are advantageous.

Capsule coating concepts have been developed over the last years aiming at the improvement of the stability during the stomach passage (US6455052B1). Coating concepts suitable for nutraceutical applications deliver the bacteria to the small intestine and disintegrate at pH 6.8. Besides the acidic conditions in the stomach, bile salts present in the small intestine additionally affect the viability of microorganisms. Therefore, coatings for solid dosage forms capable of delivering microbiota to the colon would provide additional benefits. The microbiota would be protected from the acidic conditions in the stomach as well as the bile salts in the small intestine. Furthermore, the colon displays the physiologically preferred site of delivery. An industrial applicable coating concept for the delivery of viable and culturable microbiota to the colon for nutraceutical applications is not known so far.

Therefore, it was an objective of the present invention to provide a colonic delivery system for microorganisms, which is capable of transferring viable microbiota to the colon, especially for bacteria, which are sensitive to acidic and/or aerobe environments, regardless of a fasted or fed state.

A subject of the present invention is therefore a colonic delivery system for microorganisms comprising a capsule comprising one or more bacterial or yeast strains, wherein the capsule comprises a coating comprising a polymer polymerized from 10 to 30 % by weight methyl methacrylate, 50 to 70 % by weight methyl acrylate and 5 to 15 % by weight methacrylic acid.

US5644011 describes a coating and binder composition for pharmaceutical agents. The coating or binder is a (meth)acrylate copolymer produced by emulsion polymerization in the form of an aqueous dispersion and may have a composition of (A) about 10 to 25 wt% methacrylic acid, (B) about 40 to 70 wt% methyl acrylate, and (C) 20 to 40 wt% methyl methacrylate, based on a total copolymer weight of 100 wt%.

EUDRAGUARD® biotic is a well-known commercially available (meth)acrylate copolymer product for pharmaceutical applications in the form of a 30 % by weight aqueous dispersion. The copolymer is polymerized from 10 % by weight methacrylic acid, 65 % by weight methyl acrylate, and 25 % by weight methyl methacrylate and thus corresponds to US 5644011 example B2. The molecular weight is about 280,000 g/mol.

The present invention describes a delivery system capable of transferring viable microbiota to the colon. The targeted colonic delivery system is of particular relevance for bacteria or yeasts, which are sensitive to acidic and/or aerobe environments, regardless of a fasted or fed state. The present invention refers to the targeted colon delivery of bacterial or yeast strains, which are sensitive at a pH range of 1.0-6.8, corresponding to the GIT tract comprised between stomach and upper small intestine. The same invention also prevents these bacterial or yeast strains from being damaged through the bile salts produced in the upper small intestine.

To achieve colonic delivery, microorganisms were encapsulated and a coating was applied, which starts to disintegrate at pH >7.0. Viable and culturable bacteria of sensitive species were found in simulated large intestine conditions (pH 7.5) upon release. Surprisingly, viable and culturable bacteria of sensitive species could be retained by not fully disintegrated capsules after passing the small intestine (within the used test system) as well, corroborating the unique protective property of the coating. Consequently, inherent metabolic activity could be detected within 24 h of colonic incubation.

On the other hand, the same microorganisms were released from the uncoated capsules in the early stage of the simulated stomach condition upon complete disintegration; during the passage along the simulated GIT they encountered a pH range of between 1.0-7.0 and bile salts. Consequently, they lost their viability and no or at least significantly reduced metabolic activity was detected within 24 h of colonic incubation.

In another experiment, a coating was applied which starts disintegrating at the early stage of the small intestine (pH 5.5). The bacteria lost their viability upon release and no or at least significantly reduced metabolic activity occurred within 24 h of colonic incubation.

The delivery system according to the present invention comprises a capsule as solid dosage form, consisting of hydroxypropyl methylcellulose (HPMC), starch or gelatin, filled with bacteria embedded in a dry powder mixture. The hard capsule gap is either sealed with a subcoat or via a banding procedure. To achieve the delivery of bacteria to the colon, a coating based on EUDRAGUARD® biotic is applied to the capsule, which is capable of starting to disintegrate at pH conditions in the lower small intestine and the colon (pH > 7.0).

The capsule does not disintegrate during in vitro experiments simulating the stomach (pH 1.2) and the small intestine (pH 6.8). It disintegrates under simulated colonic conditions within 1 h. Viable and culturable bacteria of sensitive species can be detected in simulated large intestine conditions upon release. Surprisingly, viable and culturable bacteria of highly sensitive species can be retained, corroborating the unique protective property of the coating. Consequently, inherent metabolic activity can be detected during colonic incubation over 24 h.

For comparison, the same bacteria composition is already released from uncoated capsules in the early stage of the simulated stomach condition upon complete disintegration of the capsule. During the passage along the simulated gastrointestinal tract the bacteria encounter pH conditions between 1.0-7.0 as well as bile salts. Consequently, they lose their viability and no metabolic activity is detected within 24 h of colonic incubation.

In an advantageous configuration, the polymer is polymerized from 20 to 30 % by weight methyl methacrylate, 60 to 70 % by weight methyl acrylate and 8 to 12 % by weight methacrylic acid. It is further preferred, when the polymer is polymerized from 25 % by weight methyl methacrylate, 65 % by weight methyl acrylate and 10 % by weight methacrylic acid.

In an alternative embodiment of the present invention, the capsule is sealed by a subcoat or encircling band and wherein the capsule and/or the sealing material are made from one of the following materials: Hydroxypropyl methylcellulose (HPMC), gelatin, pullulan, starch, a water-soluble polyvinyl derivative, a polyethylene glycol or a copolymer of C1- to C4-alkylester of (meth)acrylic acid and (meth)acrylic acid, preferably HPMC or gelatin.

In a preferred embodiment of the present invention, the bacterial or yeast strain is a probiotic strain selected from: *Bifidobacterium, Carnobacterium, Corynebacterium, Lactobacillus, Lactococcus, Leuconostoc, Microbacterium, Oenococcus, Pasteuria, Pediococcus, Propionibacterium, Streptococcus, Bacillus, Geobacillus, Gluconobacter, Xanthonomas, Candida, Debaryomyces, Hanseniaspora, Kluyveromyces, Komagataella, Lindnera, Ogataea, Saccharomyces, Schizosaccharomyces, Wickerhamomyces, Xanthophyllomyces, Yarrowia.*

The colonic delivery system according to the present invention is preferably used for the probiotic strain *B. bifidum.* In an alternative embodiment, the probiotic strains are selected from: *L. acidophilus, L. casei, L. rhamnosus, L. reuteri, B. bifidum, S. thermophilus.*

In a further preferred configuration, the colonic delivery systems comprises a combination of the probiotic strains *L. acidophilus LA 13100, L. casei LA 13120, L. rhamnosus LA 13220, L. reuteri LA 13210, B. bifidum LA 13040* and S. *thermophilus LA 13290.*

In a further alternative embodiment of the present invention, the colonic delivery system contains at least one further ingredient selected from proteins, carbohydrates, fats, further probiotics, prebiotics, enzymes, vitamins, immune modulators, milk replacers, minerals, amino acids, coccidiostats, acid-based products, plant-based products, medicines, and combinations thereof.

A further subject of the present invention is the use of a colonic delivery system according to the present invention as a food supplement.

Another subject of the present invention is the use of a colonic delivery system according to the present invention as a probiotic ingredient in food products.

A further subject of the present invention is a pharmaceutical composition containing a colonic delivery system according to the present invention.

A colonic delivery system for improving the health status, in particular gut health, cardiovascular health, healthy weight management or immune health of a human being is a further subject of the present invention.

### Additional Information:

### Capsules:

According to the present invention, the sealed capsule may be any capsule wherein the two parts of the capsule are sealed together in order to prevent accidental separation and access of air and moisture. Typically, the sealing may be achieved by providing a subcoat or encircling band. Capsule and sealing may preferably be made from the same material, however, embodiments where this is not the case are also comprised by the present invention.

Capsule and/or sealing may be made from the following materials: HPMC, gelatin, pullulan, starch, methylcellulose, a water-soluble polyvinyl derivative such as polyvinylpyrrolidone or polyvinyl alcohol, a polyethylene glycol; a copolymer of C1- to C4-alkylester of (meth)acrylic acid and (meth)acrylic acid, such as a polymer being polymerized from 10 to 30 % by weight methyl methacrylate, 50 to 70% by weight methyl acrylate and 5 to 15% by weight methacrylic acid. In another preferred embodiment of the present invention, the sealing is composed of an acetylated pre-gelled starch containing at least 50 wt% of amylose and having a percentage of acetylic groups of 0.5 wt% to 2.5 wt%. In another preferred embodiment of the present invention, the sealing of the sealed capsule exhibits a surface density of 5 mg/cm² - 15 mg/cm².

The sealing may contain an anti-tacking agent and/or plasticizer in the same type and amounts as functional coating. The sealing level is 2-50 mg/cm², preferably 5-15 mg/cm².

Sealed capsules may be coated with a standard drum coater, fluid bed coater or dragee coating pan under controlled flow conditions as described in Pharmazeutische Technologie, Bauer, Frömming, Führer, 9.Auflage (ISBN 978-3-8047-2552-2), or similar technologies.

### Functional coating composition

### Emulsifier

In the context of the present invention, suitable emulsifier components may be selected from the following: Polysorbates (Tween® series), polyoxyethylated glycol monoethers (like the Brij® series), polyoxyethylated alkyl phenols (like the Triton® series or the Igepal series). In preferred embodiments of the present invention, amphiphilic emulsifier components are selected from the following: Polysorbate 80, Poloxamer 188.

### Plasticizer

Plasticizers can be selected from hydrophilic substances, such as glycerin, polyethylene glycols, polyethylene glycol monomethyl ester, propylene glycol or sorbitol sorbitan solutions, or hydrophobic substances, such as acetyl tributyl citrate, acetyl triethyl citrate, castor oil, diacetylated monoglycerides, dibutyl sebacate, diethyl phthalate, triacetin, tributyl citrate, triethyl citrate.

The plasticizer is applied in general amounts of 0-50 % on polymer, preferably 1-10 % on polymer. The plasticizer can be included in the sealing formulation.

### Anti-Tacking Agent

In the context of the present invention, suitable anti-tacking agents include talc, glyceryl monostearate, kaoline, fumed silica, precipitated silica, magnesium stearate, calcium stearate, zinc stearate, alcaline earth metal salts, alkaline metal salts. In preferred embodiments of the present invention, anti-tacking agents are selected from the following: talc and glyceryl monostearate.

The anti-tacking agent is applied in general amounts of 0-400 % on polymer, preferably 25-100 %. The anti-tacking agent can also be included in the sealing formulation.

### Functional Polymer

The Polymer as disclosed is polymerized from 10 to 30 % by weight methyl methacrylate, 50 to 70 % by weight methyl acrylate and 5 to 15 % by weight methacrylic acid.

The polymer dispersion as disclosed may preferably comprise 15 to 50 % by weight of a polymer polymerized from 20 to 30 % by weight methyl methacrylate, 60 to 70 % by weight methyl acrylate and 8 to 12 % by weight methacrylic acid. Most preferred the polymer is polymerized from 25 % by weight methyl methacrylate, 65 % by weight methyl acrylate and 10 % by weight methacrylic acid.

A 30 % by weight aqueous dispersion of a polymer polymerized from 25 % by weight methyl methacrylate, 65 % by weight methyl acrylate and 10 % by weight methacrylic acid corresponds to the commercial product EUDRAGUARD® biotic.

The percentages of the monomers add up to 100 %.

The functional polymer is applied in amounts of 2-30 mg/cm², preferably 5-20 mg/cm².

### Microorganisms:

Capsule filling is comprising one or more microorganism(s), either as single strain or mixture of strains.

The microorganisms are preferably selected from the following probiotic bacterial strains:
*Bifidobacterium adolescentis, Bifidobacterium animalis, Bifidobacterium bifium, Bifidobacterium beve, Bifidobacterium longum, Cornobacterium divergens, Corynebacterium glutamicum, Lactobacillus acidophilus, Lactobacillus amylolyticus, Lactobacillus amylovorus, Lactobacillus animalis, Lactobacillus alimentarius, Lactobacillus aviaries, Lactobacillus brevis, Lactobacillus buchneri, Lactobacillus casei, Lactobacillus cellobiosus, Lactobacillus collinoides, Lactobacillus coryniformis, Lactobacillus crispatus, Lactobacillus curvatus, Lactobacillus delbrueckii, Lactobacillus diolivorans, Lactobacillus farciminis, Lactobacillus fermentum, Lactobacillus gallinarum, Lactobacillus gasseri, Lactobacillus helveticus, Lactobacillus hilgardii, Lactobacillus johnsonii, Lactobacillus kefiranofaciens, Lactobacillus kefiri, Lactobacillus mucosae, Lactobacillus panis, Lactobacillus paracasei, Lactobacillus paraplantarum, Lactobacillus pentosus, Lactobacillus plantarum, Lactobacillus pontis, Lactobacillus reuteri, Lactobacillus rhamnosus, Lactobacillus sakei, Lactobacillus salivarius, Lactobacillus sanfranciscensis, Lactococcus lactis, Leuconostoc citreum, Leuconostoc lactis, Leuconostoc mesenteroides, Leuconostoc pseudomesenteroides, Microbacterium imperial, Oenococcus oeni, Pasteuria nishizawae, Pediococcus acidilactici, Pediococcus dextrinicus, Pediococcus parvulus, Pediococcus pentosaceus, Propionibacterium acidipropionici, Propionibacterium freudenreichii, Streptocuccus thermophiles, Bacillus myloliquefaciens, Bacillus atrophaeus, Bacillus clausii, Bacillus flexus, Bacillus fusiformis, Bacillus lentus, Bacillus licheniformis, Bacillus megaterium, Bacillus mojavensis, Bacillus pumilus, Bacillus smithii, Bacillus subtilis, Bacillus vallismortis, Geobacillus stearothermophilus, Gluconobacter oxydans, Xanthomonas campestris,*
or from the following yeasts: *Candida cylindracea, Debaryomyces hansii, Hanseniaspora uvarum, Kluyveromyces lactis, Kluyveromyces marxianus, Komagataella pastoris, Komagataella phaffi, Lindnera jadinii, Ogataea angusta, Saccharomyces bayanus, Saccharomyces cerevisiae, Saccharomyces pastorianus, Schizosaccharomyces pombe, Wickerhamomyces anomalus, Xanthophyllomaces dendrorhous, Yarrowia lipolytica.*

### Working Examples

Testing system: Simulator of the Human Intestinal Microbial Ecosystem (SHIME®).

The used reactor setup is an adaptation of the SHIME® system described by Molly at al (1993) and stimulates within the same reactor the physiological conditions that are representative of the human GIT both under fed and fasted conditions over time; this includes addition of specific enzymes and nutritional media as well as dynamic pH profiles to mimic the in vivo condition more closely. In fed conditions stomach pH has a start value of 5.5 (ST0) and drops down to a value of 2.0 over 2 h (ST2), corresponding to the average resident time of a capsule in fed stomach. In the small intestine, the pH is at 6.5 at time point 180 min (SI1) and gradually increases to pH 7.0 after 240 min (SI2) and up to pH 7.5 after 300 min (SI3).

In fasted conditions instead, gastric pH has a permanent value of 2.0 (ST0), however, the average resident time is 45 min. Upon entering the small intestine, the capsule encounters a pH of 5.5 which gradually increases from pH 6.5 after 120 min (SI1) and pH 7.0 after 180 min (SI2) up to pH 7.5 after 240 min (SI3). After passage of the GI tract, the metabolic activity during colonic incubation is analyzed.

Examples 1-3 were performed under fed conditions.

Examples 4-5 were conducted under fasted conditions.

### Example 1 (comparative example): Capsule containing probiotic strains

Capsule type: HPMC, size 3, without coating
Capsule content: Nutriose® FM (maize starch), *L. plantarum LA13200, B. bifidum LA 13040*
Total capsule weight: -195 mg
Total bacterial count: -9,7 log10CFU/capsule

### Results

Capsules fully disintegrated during gastric simulation, where the bacteria were released. As such, viability of *B. bifidum* and *L. plantarum* went lost immediately. No metabolic activity was detected for *B. bifidum,* and only low metabolic activity was detected for *L. plantarum.*

**Table 1: Bacterial count during GIT simulation in SHIME®**

| Sampling point | Time point [min] | pH | *L. plantarum L 13200* [log₁₀CFU] | *B. bifidum L13040* [log₁₀CFU] |
|---|---|---|---|---|
| Stomach ST0 | 0 | 5.5 | 5.36 | 5.26 |
| Stomach ST2 | 120 | 2.0 | 0.00 | 0.00 |
| Intestine SI1 | 180 | 6.5 | 0.00 | 0.00 |
| Intestine SI2 | 240 | 7.0 | 0.00 | 0.00 |
| Intestine SI3 | 300 | 7.5 | 0.00 | 0.00 |

**Table 2: Quantification of metabolic activity during colonic incubation after GIT passage: total lactate (L. plantarum) and total acetate (B. bifidum)**

| Sampling point | Total lactate [mM] | Total acetate [mM] |
|---|---|---|
| 1 h | 0.1 | 0.00 |
| 3 h | 0.1 | 0.00 |
| 24 h | 2.1 | 0.00 |

### Example 2 (comparative example): Capsule containing probiotic strains coated with maize starch

Capsule type: HPMC, size 3, with starch coating
Capsule content: Nutriose® FM, *L. plantarum LA13200, B. bifidum LA 13040*
Total capsule weight: -195 mg
Total bacterial count: -9.7 log10CFU/capsule
Batch size: 500 g

**Table 3 Coating composition**

| Compound | Dry substance [g] | Content based on coating [%] | Weight gain [%] | Content based on capsule [%] |
|---|---|---|---|---|
| EUDRAGUARD® natural | 111.4 | 58.8 | 22.3 | 16.2 |
| Glycerin | 22.3 | 11.8 | 4.5 | 3.2 |
| Talc | 55.7 | 29.4 | 11.1 | 8.1 |

Coating equipment: O'Hara Labcoat 1 drum coater
Coating parameters
- Product temperature [°C]: 25-27
- Inlet air volume [m³/h]: 100
- Inlet air temperature [°C]: 30-37
- Atomizing pressure [bar]: 1.0
- Spray rate [g/min]: 3-7

### Results

Capsules were intact during gastric simulation and fully disintegrated at the early stage of the simulated small intestine (pH 5.5), where the bacteria were released. As such, viability of *B. bifidum* went lost immediately and no metabolic activity was detected, whereas *L. plantarum* showed both high viability and metabolic activity.

**Table 3: Bacterial count during GIT simulation in SHIME®**

| Sampling point | Time point [min] | pH | *L. plantarum L 13200* [log₁₀CFU] | *B. bifidum L13040* [log₁₀CFU] |
|---|---|---|---|---|
| Stomach ST0 | 0 | 5.5 | 0.00 | 0.00 |
| Stomach ST2 | 120 | 2.0 | 0.00 | 0.00 |
| Intestine SI1 | 180 | 6.5 | 8.30 | 0.00 |
| Intestine SI2 | 240 | 7.0 | 8.58 | 0.00 |
| Intestine SI3 | 300 | 7.5 | 8.43 | 0.00 |

**Table 4: Quantification of metabolic activity during colonic incubation after GIT passage: total lactate (L. plantarum) and total acetate (B. bifidum)**

| Sampling point | Total lactate [mM] | Total acetate [mM] |
|---|---|---|
| 1 h | 0.3 | 00 |
| 3 h | 0.0 | 0.0 |
| 24 h | 9.8 | 0.0 |

### Example 3 (according to the invention): Coated capsule containing probiotic strains

Capsule type: HPMC, size 3, with (meth)acrylic coating
Capsule content: Nutriose® FM (maize starch), *L. plantarum LA13200, B. bifidum LA 13040* Total capsule weight: ∼195 mg
Total bacterial count: ∼9.7 log10CFU/capsule
Batch size: 500g

**Table 5: Coating composition**

| Compound | Dry substance [g] | Content based on coating [%] | Weight gain [%] | Content based on capsule [%] |
|---|---|---|---|---|
| EUDRAGUARD® biotic | 40.8 | 36.9 | 8.2 | 6.7 |
| HPMC | 43.1 | 39.0 | 8.6 | 7.1 |
| Talc | 20.4 | 18.4 | 4.0 | 3.3 |
| Polyethylene glycol | 4.3 | 3.9 | 0.9 | 0.7 |
| Triethyl citrate | 2.0 | 1.8 | 0.4 | 0.3 |

Coating equipment: O'Hara Labcoat 1 drum coater Coating parameters
- Product temperature [°C]: 24-26
- Inlet air volume [m³/h]: 100
- Inlet air temperature [°C]: 28-34
- Atomizing pressure [bar]: 1.0-1.2
- Spray rate [g/min]: 1.4-6.5

### Results

Capsules were intact during passage along stomach and small intestine simulation and disintegrated at the late stage of the large intestine (pH 7.5). As such, *L. plantarum* was found in viable and culturable state and showed high metabolic activity, and a fraction of *B. bifidum* was also found in a viable and culturable state showing inherent metabolic activity.

**Table 6: Bacterial count during GIT simulation in SHIME®**

| Sampling point | Time point [min] | pH | *L. plantarum LA 13200* [log₁₀CFU] | *B. bifidum L13040* [log₁₀CFU] |
|---|---|---|---|---|
| Stomach ST0 | 0 | 5.5 | 0.00 | 0.00 |
| Stomach ST2 | 120 | 2.0 | 0.00 | 0.00 |
| Intestine SI1 | 180 | 6.5 | 6.34 | 0.00 |
| Intestine SI2 | 240 | 7.0 | 6.55 | 0.00 |
| Intestine SI3 | 300 | 7.5 | 9.04 | 5.63 |

**Table 7: Quantification of metabolic activity during colonic incubation after GIT passage: total lactate (L. plantarum) and total acetate (B. bifidum)**

| Sampling point | Total lactate [mM] | Total acetate [mM] |
|---|---|---|
| 1 h | 0.1 | 0.0 |
| 3 h | 0.3 | 0.0 |
| 24 h | 9.4 | 2.1 |

### Example 4 (comparative example) Capsule containing probiotic strains

Capsule type: HPMC, size 3, without coating
Capsule content: Nutriose® FM (maize starch), Vitamin B2, *L. acidophilus LA 13100, L. casei LA 13120, L. rhamnosus LA 13220, L. reuteri LA 13210, B. bifidum LA 13040, S. thermophilus LA* 13290. This bacteria composition has been selected to improve the overall stability compared to Examples 1-3.

Total capsule weight: -217 mg
Total bacterial count: -9.7 log10CFU/capsule

### Results

Capsules fully disintegrated during the gastric simulation, where the bacteria were released. As such, viability of bacteria went lost immediately. Due to the improved overall stability of the mixture, metabolic activity was detected for *Lactobacilli* and *Streptococci,* and residual metabolic activity was also detected for *B. bifidum.*

**Table 8: Bacterial count during GIT simulation in SHIME®**

| Sampling point | Time point [min] | pH | *Total Lactobacilli* [log₁₀CFU] | *S. termophilus LA 13290* [log₁₀CFU] | *B. bifidum L13040* [log₁₀CFU] |
|---|---|---|---|---|---|
| Stomach ST0 | 0 | 2.0 | 0.00 | 0.00 | 0.00 |
| Stomach ST45 | 45 | 2.0 | 6.82 | 6.00 | 0.00 |
| Intestine SI1 | 120 | 6.5 | 0.00 | 0.00 | 0.00 |
| Intestine SI2 | 180 | 7.0 | 0.00 | 0.00 | 0.00 |
| Intestine SI3 | 240 | 7.5 | 0.00 | 0.00 | 0.00 |

**Table 9: Quantification of metabolic activity during colonic incubation after GIT passage: total lactate (Lactobacilli and S. thermophilus) and total acetate (B. bifidum)**

| Sampling point | Total lactate [mM] | Total acetate [mM] |
|---|---|---|
| 24 h | 4.37 | 1.18 |

### Example 5 (according to the invention): Coated capsule containing probiotic strains

Capsule type: HPMC, size 3, with (meth)acrylic coating
Capsule content: Nutriose® FM (maize starch), Vitamin B2, *L. acidophilus LA 13100, L. casei LA 13120, L. rhamnosus LA 13220, L. reuteri LA 13210, B. bifidum LA 13040, S. thermophilus LA* 13290. This bacteria composition has been selected to improve the overall stability compared to Examples 1-3.
Total capsule weight: -217 mg
Total bacterial count: -9.7 log10CFU/capsule
Batch size: 27.020 g

**Table 10: Coating composition**

| Compound | Dry substance [g] | Content based on coating [%] | Weight gain [%] | Content based on capsule [%] |
|---|---|---|---|---|
| EUDRAGUARD® biotic | 2998.4 | 46.5 | 11.1 | 9.0 |
| HPMC | 1155.4 | 17.9 | 4.3 | 3.6 |
| Talc | 2077.0 | 32.2 | 7.7 | 6.2 |
| Polyethylene glycol | 115.5 | 1.8 | 0.4 | 0.4 |
| Triethyl citrate | 104.9 | 1.6 | 0.4 | 0.3 |

Coating equipment: IMA EFFECTA 200
Coating parameters
- Product temperature [°C]: 27-29
- Inlet air volume [m³/h]: 4.000
- Inlet air temperature [°C]: 37-40
- Atomizing pressure [bar]: 1.0
- Spray rate [g/min]: 180-200

### Results

Capsules were damaged during the last stage of the small intestine simulation and did not fully disintegrate after passing the small intestine, but remained partly intact. Hence, most of the Lactobacilli and S. *termophilus* were retained by the capsule in a viable and culturable state; in case of *B. bifidum* only the fraction retained by the capsule was found in a viable and culturable state and a significant acetate production occurred within 24 h colonic incubation.

**Table 11: Bacterial count during GIT simulation in SHIME®**

| Sampling point | Time point [min] | pH | Total *Lactobacilli* [log₁₀CFU] | *S. termophilus LA13290* [log₁₀CFU] | *B. bifidum L13040* [log₁₀CFU] |
|---|---|---|---|---|---|
| Stomach ST0 | 0 | 2.0 | 0.00 | 0.00 | 0.00 |
| Stomach ST45 | 45 | 2.0 | 0.00 | 0.00 | 0.00 |
| Intestine SI1 | 120 | 6.5 | 5.01 | 5.24 | 0.00 |
| Intestine SI2 | 180 | 7.0 | 5.91 | 5.96 | 0.00 |
| Intestine SI3 | 240 | 7.5 | 6.08 | 6.07 | 0.00 |
| Capsule not disintegrated at the end of small intestine | 240 | -- | 8.61 | 8.46 | 5.71 |

**Table 12: Quantification of metabolic activity during colonic incubation after GIT passage: total lactate (Lactobacilli and S. thermophilus) and total acetate (B. bifidum)**

| Sampling point | Total lactate [mM] | Total acetate [mM] |
|---|---|---|
| 24 h | 13.79 | 5.13 |

## Claims

1. A colonic delivery system for microorganisms comprising
a capsule comprising one or more bacterial or yeast strains,
**characterized in that** the capsule comprises a coating comprising a polymer polymerized from 10 to 30 % by weight methyl methacrylate, 50 to 70 % by weight methyl acrylate and 5 to 15 % by weight methacrylic acid.

2. Colonic delivery system according to claim 1, wherein the polymer is polymerized from 20 to 30 % by weight methyl methacrylate, 60 to 70 % by weight methyl acrylate and 8 to 12 % by weight methacrylic acid.

3. Colonic delivery system according to any one of the preceding claims, wherein the polymer is polymerized from 25 % by weight methyl methacrylate, 65 % by weight methyl acrylate and 10 % by weight methacrylic acid.

4. Colonic delivery system according to any one of the preceding claims, wherein the capsule is sealed by a subcoat or encircling band and wherein the capsule and/or the sealing material are made from one of the following materials: Hydroxypropyl methylcellulose (HPMC), gelatin, pullulan, starch, a water-soluble polyvinyl derivative, a polyethylene glycol or a copolymer of C1- to C4-alkylester of (meth)acrylic acid and (meth)acrylic acid, preferably HPMC or gelatin.

5. Colonic delivery system according to any one of the preceding claims, wherein the bacterial or yeast strain is a probiotic strain selected from: *Bifidobacterium, Carnobacterium, Corynebacterium, Lactobacillus, Lactococcus, Leuconostoc, Microbacterium, Oenococcus, Pasteuria, Pediococcus, Propionibacterium, Streptococcus, Bacillus, Geobacillus, Gluconobacter, Xanthonomas, Candida, Debaryomyces, Hanseniaspora, Kluyveromyces, Komagataella, Lindnera, Ogataea, Saccharomyces, Schizosaccharomyces, Wickerhamomyces, Xanthophyllomyces, Yarrowia.*

6. Colonic delivery system according to claim 5, wherein the probiotic strain is *B. bifidum.*

7. Colonic delivery system according to claim 5, wherein the probiotic strains are selected from: *L. acidophilus LA 13100, L. casei LA 13120, L. rhamnosus LA 13220, L. reuteri LA 13210, B. bifidum LA 13040, S. thermophilus LA 13290.*

8. Colonic delivery system according to any one of the preceding claims, containing at least one further ingredient selected from proteins, carbohydrates, fats, further probiotics, prebiotics, enzymes, vitamins, immune modulators, milk replacers, minerals, amino acids, coccidiostats, plant-based products, acid-based products, medicines, and combinations thereof.

9. Use of a colonic delivery system according to any one of the preceding claims as a food supplement.

10. Use of a colonic delivery system according to any one of the preceding claims as a probiotic ingredient in food products.

11. A pharmaceutical composition containing a colonic delivery system according to any one of claims 1 to 8.

12. A colonic delivery system according to claims 1 or 8 for improving the health status, in particular gut health, cardiovascular health, healthy weight management or immune health of a human being.
